# DEMANDE DE BREVET EUROPEEN

(11) **EP 1 639 970 A2**
(43) Date de publication de la demande: **29.03.2006**
(21) Numéro de dépôt: 05356166.8
(22) Date de dépôt: 22.09.2005
(51) Int. Cl.: A61F 5/01

(54) **Orthèse de genou en textile comportant une articulation polycentrique à engrenage**

(30) Priorité: 22.09.2004 FR 0410016
(71) Demandeur: Gibaud, F-42000 Saint Etienne (FR)
(72) Inventeur: Anquetil, Lionel, 42000 Saint Etienne (FR)
(74) Mandataire: Delorme, Nicolas

(57) **Abrégé**

Cette orthèse de genou présente un élément de textile élastique tubulaire équipé d'organes de raidissement (9) latéraux parallèles comprenant chacun un montant fémoral (10a) susceptible de venir en appui sur la face latérale d'une cuisse et un montant tibial (10b) susceptible de venir en appui sur la face latérale d'une jambe, les montants (10a, 10b) étant reliés deux à deux par une biellette disposée au niveau de l'articulation du genou comprenant, à chacune de ses extrémités, un axe d'articulation (13) sur chacun desquels s'articulent respectivement le montant fémoral (10a) et le montant tibial (10b).

Selon l'invention, pour au moins l'un des organes de raidissement, le montant fémoral (10a) présente, à son extrémité en regard du montant tibial, un secteur denté (14a) engrenant avec un secteur denté (14b) ménagé dans l'extrémité du montant tibial (10b) en regard du montant fémoral.

## Description

L'invention concerne une orthèse de genou en textile comportant des articulations latérales polycentriques.

Dans le domaine de l'orthopédie, il est bien connu de prescrire le port d'orthèse de genou pour stabiliser l'articulation du genou après une entorse des ligaments latéraux ou après une rupture des ligaments croisés. Le port d'une orthèse de genou est également prescrit en cas de laxité chronique de l'articulation du genou.

Une orthèse largement prescrite présente une structure tubulaire exclusivement réalisée en textile élastique qui est renforcée latéralement par deux éléments de raidissement comprenant deux paires de montants latéraux reliés deux à deux par une articulation polycentrique.

Un montant se trouve en appui sur la face latérale de la cuisse, le second montant se trouve en appui sur la face latérale de la jambe, tandis que l'articulation polycentrique est au niveau du genou.

Par articulation polycentrique, on entend une articulation comprenant deux axes de rotation. Pour réaliser une telle articulation, chaque paire de montants s'articule sur une biellette pourvue à chacune de ses extrémités d'un axe d'articulation.

Une telle orthèse est notamment décrite dans le document FR A-2 720 270.

Ce type d'orthèse donne globalement satisfaction sauf sur un point.

En effet, la stabilisation de l'articulation est assurée essentiellement par la nature fortement élastique du textile dans lequel l'orthèse est réalisée.

Dans le plan frontal, la stabilisation est renforcée par les montants latéraux. En revanche, dans le plan antéro postérieur, on observe dans le cas d'orthèse renforcée par des articulations polycentriques, un mouvement de tiroir antéro postérieur, c'est-à-dire une mobilité antéro postérieure anormale qui ne s'avère pas être contenue pour la seule propriété de contention de tissu élastique. On doit rappeler qu'il est essentiel de prévenir ce mouvement de tiroir qui est normalement limité par les ligaments croisés antérieur et postérieur. Or, le port de ce type d'orthèse est précisément prescrit pour des sujets présentant une laxité dans le plan antéro postérieur.

Un but de l'invention est donc de proposer une orthèse en textile élastique renforcée latéralement qui limite le mouvement de tiroir antéro postérieur.

L'invention a essentiellement pour objet une orthèse de genou présentant un élément tubulaire en matière textile élastique équipé d'organes de raidissement latéraux parallèles comprenant chacun un montant fémoral susceptible de venir en appui sur la face latérale d'une cuisse et un montant tibial susceptible de venir en appui sur la face latérale d'une jambe, les montants étant reliés deux à deux par une biellette disposée au niveau de l'articulation du genou comprenant, à chacune de ses extrémités, un axe d'articulation sur chacun desquels s'articulent respectivement le montant fémoral et le montant tibial, caractérisée en ce que, pour au moins l'un des organes de raidissement, le montant fémoral présente, à son extrémité en regard du montant tibial, un secteur denté engrenant avec un secteur denté ménagé dans l'extrémité du montant tibial en regard du montant fémoral.

Ainsi, lors des mouvements de flexion et extension du genou, le montant fémoral et le montant tibial de l'organe de raidissement latéral sont constamment en prise, ce qui interdit un mouvement du tiroir antéro postérieur, c'est-à-dire un mouvement dans le plan antéro postérieur de translation du tibia par rapport au fémur et non de rotation pure. Grâce à cet engrènement du montant fémoral et du montant tibial, la stabilité procurée par l'orthèse en textile selon l'invention est considérablement accrue.

Selon une forme de réalisation préférée, le secteur denté de chacun des montants s'étend sur un angle d'environ 170°.

De façon avantageuse, chaque montant présente un coude à son extrémité de liaison.

Pour améliorer la rigidité des montants, chacun des montants présente une nervure de renfort longitudinale.

Selon une possibilité, deux biellettes prennent en sandwich les extrémités du montant fémoral et du montant tibial.

Dans une forme de réalisation préférée, les deux moyens de raidissement sont glissés dans deux fourreaux rapportés sur la face extérieure de l'élément de textile tubulaire.

Par ailleurs, l'élément tubulaire présente une ouverture antérieure dégageant l'articulation rotulienne et présente une ouverture postérieure dégageant le creux poplité.

Pour assurer un meilleur serrage de l'orthèse, elle est munie d'au moins une sangle de serrage permettant d'assurer le serrage de l'orthèse sur la cuisse et au moins une sangle de serrage permettant d'assurer le serrage sur la jambe.

Pour sa bonne compréhension, l'invention est décrite en référence au dessin ci annexé représentant, à titre d'exemple non limitatif, une forme de réalisation d'une orthèse de genou selon l'invention.

Figure 1 montre, en vue de face, l'orthèse de genou, celle-ci étant représentée à plat,

Figure 2 montre un organe de raidissement de cette orthèse,

Figure 3 est une vue en coupe selon III-III de figure 2.

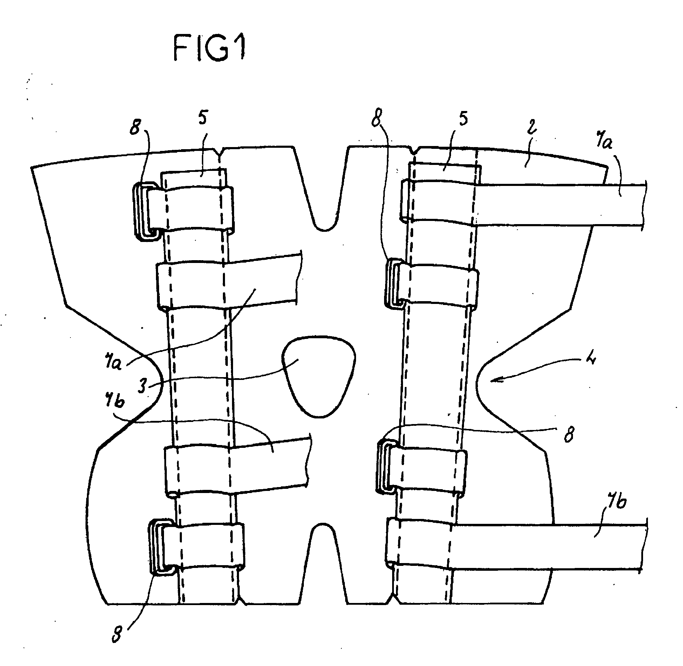

L'orthèse, objet de la présente invention, présente comme le montre la figure 1 un élément de textile 2 élastique. Sur le dessin, cet élément de textile 2 est représenté à plat mais il présente une forme tubulaire qui lui permet d'être enfilé sur le membre inférieur d'un patient et peut ainsi venir se placer au niveau de l'articulation du genou.

Cet élément de textile 2 tubulaire est muni de deux fourreaux 5. Ces deux fourreaux 5, qui sont cousus sur l'élément textile tubulaire, sont disposés sur les faces latérales de l'orthèse. On peut noter que chacun de ces fourreaux s'étend sur toute la hauteur de l'orthèse.

Comme on peut le voir, l'élément de textile 2 présente une ouverture antérieure 3 destinée à dégager l'articulation rotulienne et une ouverture postérieure 4 destinée à dégager le creux poplité du genou.

Ces deux ouvertures antérieure 3 et postérieure 4 définissent un plan qui sépare une partie fémorale de l'orthèse qui vient en recouvrir la cuisse d'un patient et une partie tibiale qui vient en recouvrir la jambe d'un patient.

Les sangles de serrage 7 sont disposées symétriquement par rapport au plan passant par les ouvertures postérieure 3 et antérieure 4 et comprennent donc une sangle de serrage frontale qui est fixée sur l'un des fourreaux et une sangle de serrage postérieure qui est fixée sur l'autre fourreau. Pour le serrage, chacune de ces sangles postérieure et antérieure passe dans un anneau 8 fixé sur le fourreau opposé.

Le blocage de ces sangles s'effectue par un élément de textile auto agrippant.

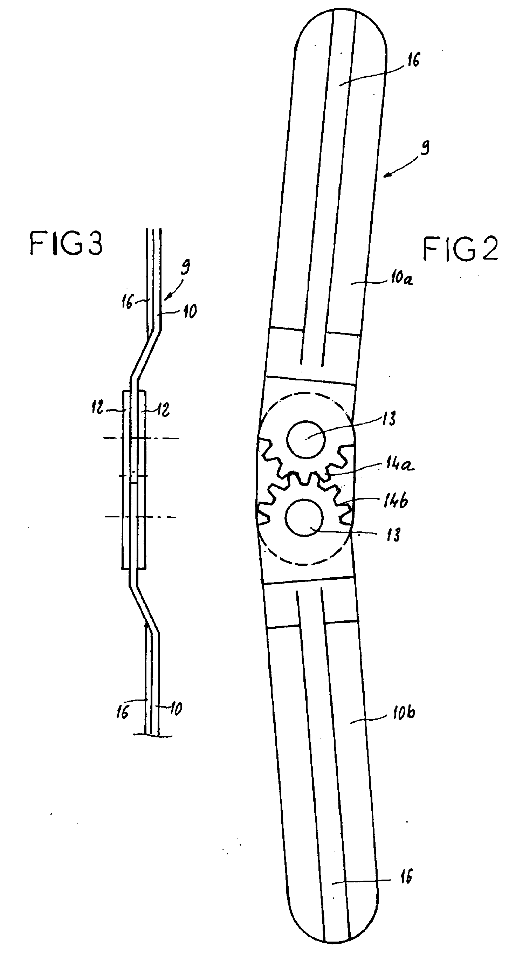

A l'intérieur de chacun de ces fourreaux 5 est disposé un organe de raidissement 9 qui est représenté plus clairement aux figures 2 et 3.

En se référant tout d'abord à la figure 2, on peut voir que l'organe de raidissement 9 est constitué de deux montants 10 fémoral et tibial reliés l'un à l'autre par deux biellettes 12. Ces biellettes 12 sont pourvues de deux axes de rotation dans lesquels s'engage chacun des montants 10 qui, pour cela, sont équipés d'un perçage. La liaison des deux montants est réalisée par ces biellettes 12 parallèles qui prennent en sandwich les extrémités des deux montants 10, comme on peut le voir sur la figure 3.

On note que, sur la figure 2, l'une des biellettes 12 a été ôtée pour permettre d'illustrer la caractéristique essentielle de l'invention qui est que les montants 10 sont pourvus chacun d'un secteur denté 14.

Comme on peut le voir très clairement à la figure 2, les montants 10 fémoral et tibial s'engrènent l'un dans l'autre au niveau de leur articulation. Ainsi, les montants ne sont pas indépendants l'un de l'autre mais sont réunis, non seulement par les biellettes 12, mais aussi par les secteurs dentés 14 qui s'engrènent. Le secteur denté s'étend sur un angle de 170° pour chacun des montants.

Parmi les autres caractéristiques de l'organe de raidissement 9 que l'on peut citer, on note que chacun des montants 10 est pourvu d'une nervure de raidissement 16 et on constate, en outre que, au niveau de l'articulation, chacun des montants présente un coude qui fait que l'articulation des montants 10 se trouve dans un plan parallèle décalé par rapport au plan des montants 10 eux-mêmes.

Lorsqu'un patient est appareillé d'une orthèse telle qu'elle vient d'être décrite, et que cette orthèse est serrée sur sa cuisse et sa jambe par les sangles de serrage 7, l'articulation de genou du patient est stabilisée par, bien entendu, la nature fortement élastique du textile. Cette stabilisation est également renforcée dans le plan frontal par les organes de raidissement 9. Lors de mouvement de flexion/extension du genou, le mouvement de tiroir antéro postérieur qui peut se produire en cas de faiblesse des ligaments croisés antérieurs et postérieurs est jugulé par l'action conjuguée des montants 10 fémoral et tibial.

En effet, ceux-ci étant engrenés, ils ne peuvent autoriser en aucun cas un mouvement parasite de translation dans le plan antéro postérieur du tibia par rapport au fémur.

Il s'agit là d'un avantage tout à fait essentiel conféré par l'invention par rapport aux orthèses de l'art antérieur.

Bien entendu, l'invention n'est pas limitée à la forme de réalisation décrite ci-dessus à titre d'exemple, mais elle en embrasse au contraire toutes variantes de réalisation.

## Revendications

1. Orthèse de genou présentant un élément de textile (2) élastique tubulaire équipé d'organes de raidissement (9) latéraux parallèles comprenant chacun un montant fémoral (10a) susceptible de venir en appui sur la face latérale d'une cuisse et un montant tibial (10b) susceptible de venir en appui sur la face latérale d'une jambe, les montants (10a, 10b) étant reliés deux à deux par une biellette (12) disposée au niveau de l'articulation du genou comprenant, à chacune de ses extrémités, un axe d'articulation (13) sur chacun desquels s'articulent respectivement le montant fémoral (10a) et le montant tibial (10b), **caractérisée en ce que**, pour au moins l'un des organes de raidissement, le montant fémoral (10a) présente, à son extrémité en regard du montant tibial, un secteur denté (14a) engrenant avec un secteur denté (14b) ménagé dans l'extrémité du montant tibial (10b) en regard du montant fémoral.

2. Orthèse du genou selon la revendication 1, **caractérisée en ce que** le secteur denté de chacun des montants s'étend sur un angle d'environ 170°.

3. Orthèse de genou selon la revendication 1 ou la revendication 2, **caractérisée en ce que** chaque montant (10a, 10b) présente un coude à son extrémité de liaison.

4. Orthèse de genou selon l'une des revendications 1 à 3, **caractérisée en ce que** chacun des montants (10a, 10b) présente une nervure (16) de renfort longitudinale.

5. Orthèse de genou selon l'une des revendications 1 à 4, **caractérisée en ce que** deux biellettes (12) prennent en sandwich les extrémités du montant fémoral (10a) et du montant tibial (10b).

6. Orthèse de genou selon l'une des revendications 1 à 5, **caractérisée en ce que** les deux moyens de raidissement (9) sont glissés dans deux fourreaux rapportés sur la face extérieure de l'élément de textile (2) tubulaire.

7. Orthèse du genou selon l'une des revendications 1 à 6, **caractérisée en ce que** l'élément tubulaire (2) présente une ouverture antérieure (3) dégageant l'articulation rotulienne.

8. Orthèse de genou selon l'une des revendications 1 à 7, **caractérisée en ce que** l'élément de textile (2) tubulaire présente une ouverture postérieure (4) dégageant le creux poplité.

9. Orthèse de genou selon l'une des revendications 1 à 8, **caractérisée en ce qu'**elle est munie d'au moins une sangle de serrage (7a) permettant d'assurer le serrage de l'orthèse sur la cuisse et au moins une sangle de serrage (7b) permettant d'assurer le serrage sur la jambe.
